(19) Europäisches Patentamt
European Patent Office
Office européen des brevets

(11) Publication number: **0 357 011 B1**

(12) # EUROPEAN PATENT SPECIFICATION

(45) Date of publication of patent specification: **31.08.94** (51) Int. Cl.$^5$: **C12Q 1/68**, C07H 21/00

(21) Application number: **89115959.2**

(22) Date of filing: **30.08.89**

(54) **Detection and amplification of target nucleic acid sequences.**

(30) Priority: **30.08.88 US 238649**
**17.08.89 US 394051**

(43) Date of publication of application:
**07.03.90 Bulletin 90/10**

(45) Publication of the grant of the patent:
**31.08.94 Bulletin 94/35**

(84) Designated Contracting States:
**AT BE CH DE ES FR GB GR IT LI NL SE**

(56) References cited:
**EP-A- 336 731**
**EP-A- 0 200 362**
**WO-A-90/11369**
**GB-A- 2 169 403**
**GB-A- 2 202 328**

**PROCEEDINGS OF THE NATL. ACADEMY OF SCIENCES USA, vol. 86, April 1989; D.J. KEMP et al., pp. 2423-2427&NUM;**

(73) Proprietor: **ABBOTT LABORATORIES**
**CHAD-0377,**
**AP6D/2,**
**One Abbott Park Road**
**Abbott Park, Illinois 60064-3500 (US)**

(72) Inventor: **Laffler, Thomas G.**
**1117 N. Harvey**
**Oak Park, IL 60302 (US)**
Inventor: **Bouma, Stanley Robert**
**834 Lange Street**
**Mundelein, IL 60060 (US)**

(74) Representative: **Modiano, Guido, Dr.-Ing. et al**
**Modiano & Associati S.r.l.**
**Via Meravigli, 16**
**I-20123 Milano (IT)**

EP 0 357 011 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

1. Field of the Invention

This invention relates to a method for detecting and amplifying a target nucleic acid sequence in a sample. In particular, this invention relates to a polymerase chain reaction which is amenable to automation. With a polymerase chain reaction (PCR), one can "amplify" (i.e., increase the number of) target oligonucleotide molecules in a sample to be analyzed for the presence of that target sequence.

2. Description of Related Art

One type of polymerase chain reaction is disclosed in U.S. Patent Numbers 4,683,202 and 4,683,195. In this type of polymerase chain reaction, two complementary polynucleotide strands are amplified by treating the strands with two oligonucleotide primers such that an extension product of each primer is synthesized which is complementary to each nucleic acid strand. The primers are selected such that the extension product of one primer forms a template for the synthesis of an extension product from the other primer once the extension product of the one primer is separated from the template. A chain reaction is maintained by a cycle of denaturing the primer extension products from their templates, treating the single-stranded molecule generated with the same primers to re-anneal, and allowing the primers to form further extension products. The cycle is repeated for as many times as it takes to increase the target nucleic acid segments to a concentration where they can be detected.

Typically, the amplified target sequence is detected by denaturing the double-stranded products formed by PCR, and treating those products with one or more reporter probes which hybridize with the extension products. The reporter probe has a detectable label, and is usually added in excess. The unhybridized reporter probe, therefore, must be separated from the hybridized reporter probe requiring a separation step. In another method of detecting the extension products without reporter probe and a separation step, the extension products are detected by gels stained with ethidium bromide.

Regardless whether reporter probes or gels are used, prior PCR methods are quite difficult to automate. Using reporter probes requires denaturing the extension products, annealing the reporter probe, and in some cases separating excess reporter probe from the reaction mixture. Using gels, of course, is time consuming, labor intensive, and thus impractical to automate if rapid results are desired.

### Summary of the Invention

The present invention is a method and kit for amplifying and detecting target nucleic acid sequences. The method involves hybridizing to each of the target and its complementary strand an oligonucleotide primer conjugated to a member of a reactive pair. In the presence of target, an extension product complementary to each nucleic acid strand is synthesized from each primer, the extension product of each primer forming a template for the synthesis of further extension products from the other primer once the extension product is separated from its complement. The primer extension products are separated from the templates on which they were synthesized to produce single-stranded molecules. The single-stranded molecules are treated with the primers under conditions that a primer extension product is synthesized using each of the single strands produced as templates to form further double-stranded products. The double stranded products are captured on a solid phase having immobilized thereon the other member of one of the reactive pairs associated with the primer so that the double-stranded products become linked to the solid phase by the one reactive pair. Before, during or after the solid phase capture step, the double stranded products are treated with the other member of the other reactive pair labeled with a detectable moiety whereby the double-stranded products are labeled with the detectable moiety. The detectable moiety associated with said solid phase is then detected to indicate whether any target was present in the original sample.

This method, therefore, provides an easy separation step using a solid phase whereby the labeled extension products can be separated from the reaction mixture at the same time that the bound label is separated from any unbound label.

The current invention also involves kits for performing DNA probe assays, which kits include primers at least one of which has a first reactive pair member linked thereto as well as solid phase materials having linked thereto a second reactive pair member reactive with the first reactive pair member.

Brief Description of the Drawings

Figure 1 is a schematic of a first embodiment of the present invention where two primers are employed, each of which is linked to a member of a reactive pair; Figure 2 is a schematic of a second embodiment of the present invention where two pairs of primers, one pair "nested" within the other, are used, the nested pair each having a reactive pair member linked thereto;

Figure 3 is a schematic of a third embodiment of the present invention where three primers are employed, one primer nested within the other two;

Figure 4 is a schematic of a fourth embodiment of the present invention where two pairs or "nested" primers are employed, one pair having one member of one reactive pair linked thereto, the other pair having one member of a second reactive pair linked thereto;

Figure 5 is a schematic of a fifth embodiment of the present invention employing two primers, one being linked to a member of a reactive pair, the other being linked to a detectable moiety;

Figure 6 is photoprint of the results of a non-instrument read assay, wherein the probes at top left contained neither biotin nor fluorescein, at top right, biotin only, at bottom left, fluorescein only, and at bottom right, both biotin and fluorescein; and

Figure 7 is a photoprint of an immunochromatographic assay, wherein the probes on strip O contained neither biotin nor fluorescein, on strip F, fluorescein only, on strip B, biotin only, and on strip BF, both biotin and fluorescein.

Detailed Description of the Invention

The current invention involves a polymerase chain reaction (PCR) using "capture" oligonucleotide primers each of which is conjugated to a member of a reactive pair. In Figure 1, a double-stranded target sequence is denatured (step A), and capture primers are annealed to the 3' end of each of the original target sequences (step B). It is important that the capture primers be of a length which does not permit one capture primer to hybridize with portions of the target nucleotide strand complementary to the other capture primer. The capture primers are each linked or conjugated at their 5' ends to a member of a reactive pair (defined below), each member being illustrated graphically by a circle or a triangle. While apparently not essential to this invention, the presence or a reactive pair at the 5' end has the additional effect of blocking any degradative reaction of 5'-3' exonucleolytic activity.

Primer extension products are then synthesized using a DNA polymerase to extend the 3' end of each of each primer (step C). The extension products are then separated from their templates by denaturing the double-stranded products of step C, and new primers are annealed to the extension products of step C (see step D). Additional extension products are then synthesized (step E). Step D is repeated using the double-stranded products of step E for as many cycles as is necessary to increase the concentration of target sequence to a level where it can be detected.

When the PCR is completed, double stranded products (step F), each strand of which has a member of a reactive pair at its 5' end represented by circles and triangles, will predominate in the reaction mixture.

Suitable reactive pair members include covalent and non-covalent reactive pairs. Suitable non-covalent reactive pairs include typical ligand-antiligand members such as antibody and antigen, biotin and avidin, enzyme and enzyme receptor, carbohydrate and lectin, enzyme and enzyme cofactors, and a pair of complementary DNA strands. It is preferred that the reactive pairs be selected from specific binding members, i.e., those members which have a non covalent specificity for one another (e.g. biotin and avidin, or antibody and antigen, or the like). Suitable covalent reactive pairs include a free thiol (attached to the 5' end of each primer) which reacts with heavy metals such as lead or mercury (attached to the solid phase or label).

The products to be detected (step F) are separated from the reaction mixture by reacting one of the reactive members (e.g., represented by the triangles) with the second member reactive or complementary with it, where the second member is conjugated or linked to a solid phase such as the microparticles shown. With the extension products attached to a solid phase, the extension products can be separated from the reaction mixture by filtering or passing through the IMx(R) disposable reaction cell sold by Abbott Laboratories.

Other solid phases can also be used. For instance, the second member can be directly linked to a medium (e.g., a membrane or filter), and the extension products passed through the medium and captured directly on it. Beads or columns can also be used in conventional solid phase separation techniques to remove the extension products from the reaction mixture, provided of course, the beads or column have the second member of the reactive pair linked thereto.

Before, during or after solid phase separation, the other reactive pair member (e.g., represented by the circle) is reacted with the second member of its reactive pair specific to it, where the second member is labeled with a detectable label as defined below. As can be seen (step H), a conjugate is formed with two complementary oligonucleotide strands each of which has at its 5' end a reactive pair member (preferably a specific binding member) which is linked via the other member of the reactive pair to either a label or a solid phase. The excess label/reactive pair member conjugate member is separated from bound label preferably by filtration using the microparticles linked to the opposite end of the DNA strand.

Once the solid phase is separated from the solution, any label associated with the solid phase indicates that primer extension products were formed using the original target nucleic acid sequence as a template. In the absence of target sequence, no extension products would be formed and the double stranded complex of step H would not be possible.

The present method of PCR is easy to automate. With oligonucleotide capture primers - each of which is conjugated to a member of a reactive pair - the primers can be processed to amplify the target sequence using conventional techniques. However, once the double-stranded products (step F) are formed, no denaturation and addition of a reporter DNA probe is necessary. The extension products can be left double-stranded, can be detected with a labeled reactive member, and separated (before, during or after detection) with a reactive member conjugated to a solid phase.

Preferably, the reactive member on one capture primer is different from the reactive member on the other capture primer. Most preferably, the two reactive members are selected from different pairs of reactive members. For instance, where the reactive member on the primer reactive with the solid phase conjugate is biotin (the solid phase conjugate including avidin or anti-biotin), the reactive member on the other primer to be conjugated with the label could be a hapten or an antigen which is reactive with a labeled antibody. Thus, the labeled reactive member will not cross react with the solid phase conjugate, and the reactive members at each end of the amplified products (step F) will not react with each other.

Alternatively, the reactive pair members conjugated or linked to each capture primer can be the same. While this configuration may be more useful in agglutination-type assays, it is less desirable for solid phase capture configurations, since it is more difficult to ensure that the double stranded complex will attach to both solid phase and label conjugate.

The method of linking or conjugating a reactive pair member to a DNA primer depends, of course, on the type of reactive pair member used. However, the linkage preferred will be a covalent bond through the 5' hydroxyl group. In one procedure, a primary aliphatic amine is added to the 5' hydroxyl of the oligonucleotide during primer synthesis via phosphoramidite chemistry. Subsequently, this amine is reacted with a hapten species (e.g. fluorescein isothiocyanate, or biotin-N-hydroxysuccinimide ester) to yield a primer linked to a reactive pair member.

Extension products are formed by exposing the primers annealed to their templates to a DNA polymerase, preferably a temperature stable DNA polymerase such as the Taq polymerase disclosed in European Patent Specification 258,017. The DNA polymerase will, of course, copy the template strand synthesizing DNA from the primers in the 5' to 3' direction. Temperature stable polymerases are preferred because the preferred way of denaturing the double stranded extension products is by exposing them to a high temperature (e.g. about 95 degrees centigrade) during the PCR cycle as disclosed in U.S. 4,683,202. However, if different methods of denaturing the extension products are used, other DNA polymerases or fragments thereof may be employed, including a Klenow fragment.

This invention can also be practiced with "nested" primers (Figure 2). A double-stranded target sequence (step A, Figure 2) is denatured, and "primary" primers are annealed to the single strands in the target region of interest. The primary primers hybridize to regions $P_2$ and $P_1$ at the 3' ends of the respective target strands. Using PCR, primary extension products are then synthesized (step C) and the process is repeated to obtain the desired degree of amplification. It is to be recognized that it does not matter how the target is first amplified in this embodiment and any method of amplification will suffice for this step.

Once the desired degree of amplification with the primary primers is complete, secondary primers (step E, Figure 2) are annealed to the primary extension products produced in steps A-D. The secondary primers are "capture" primers and have at their 5' ends members of reactive pairs as defined above. The secondary primers are synthesized so as to hybridize at regions on their respective strands near or adjacent (but not overlapping) where the 3' terminus of the primary primers would be if hybridized to the same strands. It can be seen in step E, for instance, a first secondary primer hybridizes to a sequence $P_1'$ which is adjacent the sequence $P_1$ where the first primary primer would hybridize if it were in solution with the secondary primers. A second secondary primer hybridizes at a region $P_2'$ adjacent the region $P_2$ where the second primary primer would hybridize if it were in solution.

One or more, preferably one to ten PCR cycles are performed with the secondary (capture) primers to produce double-stranded extension products (step F, Figure 2) which have reactive pair members at each end of the double-stranded molecules. These double-stranded molecules can then be detected with label conjugates and separated with solid phase conjugates as previously described.

The advantage of using nested primary and secondary primers is improved specificities. Secondary extension products will not be synthesized unless the secondary primers hybridize to the primary extension products. Thus, in the event that the primary primers hybridize to a nucleic acid sequence other than the target desired, the secondary primers will not hybridize to the actual primary extension products. The secondary extension products will only be formed if the proper primary extension products are synthesized. Accordingly, the specificity can be improved if the secondary primers are added to the reaction mixture for one or more, preferably one to ten PCR cycles to produce a detectable concentration of secondary extension products.

Instead of a pair of nested secondary primers, a single nested secondary primer can be employed (Figure 3) where a first pair of primers ($P_1$ and $P_2$) are employed to form primary extension products. One of the primary primers ($P_1$) is a "capture" primer (ie. is linked to a member of a first reactive pair), whereas the other primary primer ($P_2$) is not. A secondary "capture" primer $P_2$' (having a member of a second reactive pair at its 5' end) hybridizes to the primary extension products at a site near or adjacent (but not overlapping) the site where the 3' terminus of primer $P_2$ would hybridize if it were still in solution. A secondary extension product is formed using $P_2$' so that a double-stranded oligonucleotide sequence is formed having reactive pairs at the 5' ends of both the individual strands. (One reactive pair member comes from the primary primer and the other comes from the secondary primer.)

Alternatively, the primary primers ($P_1$ and $P_2$, Figure 4) can be linked with the same first member of a first reactive pair and the secondary primers $P_1$' and $P_2$' can be linked with a first member of a second reactive pair different from the first reactive pair. Thus, when secondary extension products are formed, hybrids such as those shown in Figure 4 having reactive pair members at the 5' ends at each strand are formed which can be separated by solid phase separation and labeled as described above. Although this embodiment is a plausible configuration, it has not been found to provide results as good as the other configurations and, therefore, is less preferred.

A fifth embodiment of this invention is illustrated in Figure 5. The double stranded target (Step A) is denatured, and a pair of primers are annealed to the strands according to the procedures described above. However, one of the primers has linked to its 5' end a directly detectable label represented by the symbol "Θ". The other primer has linked to its 5' end a member of a reactive pair as before. Extension products are synthesized (Step C), and the PCR process is repeated as many times as necessary (Step D, Figure 5) to obtain the desired degree of amplification. After amplification, the double stranded extension products are captured on a solid phase (e.g. the microparticle shown in Step E, Figure 5) which has the other member of the reactive pair (reactive with the reactive pair member on the extension products). Thus, the capture and direct detection can be accomplished simply by using one primer bearing a reactive pair member and the other primer bearing a detectable label directly attached to it.

In this fifth embodiment, it is preferred to employ a chemiluminescent or fluorescent label. The direct linkage of a chemiluminescent label to the primer can be accomplished by reacting the 5' hydroxy of the one primer during primer synthesis to a primary aliphatic amine via phosphoramidite chemistry. The primer with the amine can then be reacted with an acridinium active ester to link the acridine to the primer. The primer can also be linked directly to a fluorophore as described in Example 6 below. Either primer can then be used in PCR as described above.

Besides chemilumophores, other detectable labels can be employed including chromophores and fluorophores provided the label can survive the temperature cycling required in the PCR process.

The reactants employed in this invention can be provided in kit form to the user. The kit includes primers (at least one of which is linked with a member of a reactive pair), a DNA polymerase, and a solid phase having the other member of the reactive pair. If the first embodiment or this invention is to be practiced, the kit would include two primers, each of which is linked to members of different reactive pairs, a detectable label-reactive pair member conjugate, and the solid phase conjugate and polymerase described above. If the second or fourth embodiments are to be practiced, the kit would include two pairs of "nested primers" where at least one primer has a member of a reactive pair linked to it which member can bind to another reactive pair member linked to a solid phase also provided in the kit with DNA polymerase. If the third embodiment is to be practiced only one nested primer will be provided which primer has a reactive pair member linked to it. Finally, if the fifth embodiment is to be practiced, the kit would include two primers, one of which is linked directly to a detectable label and the other of which is attached to a member of a reactive pair. The kit would include a solid phase/reactive pair member conjugate and DNA

polymerase.

The invention will now be further described by the following examples. The examples are illustrative only and shall not be construed as limiting the invention in any way.

**Example 1: Oligonucleotide Synthesis.**

Polymerase Chain Reaction (PCR) primers designated A through G (seeTable **1** below) were synthesized on an Applied Biosystems 380B DNA synthesizer using $\beta$-cyanoethylphosphoramidites. The couplings were performed with wait times of 30 sec, in order to maximize coupling yields. After the oligonucleotide chain synthesis was complete, Aminomodifier II® (Clontech, Palo Alto, CA) was added in the last cycle to give, upon oxidation and deprotection, a primary amine-terminated oligonucleotide chain.

**Example 2: Oligonucleotide Labeling**

The PCR primers from Example 1 were linked with a member of a reactive pair essentially following the protocol of Urdea, *et. al*. [Nucleic Acids Research **16(11)** 4937-4956 (1988)].

Table 1

| Designation | Reactive Pair Member | Sequence |
|---|---|---|
| A | none | 5'-AAAATTCGCAGTCCCCAACCTCCAATCACTCACCA-3' |
| B | none | 5'-GCGGTATAAAGGGACTCACGATGCTGTACAGACTT-3 |
| C | Fl* | 5'-$NH_2$-TTGTCCTGGTTATCGCTGGATGTGTCTGCGGCGTT-3' |
| D | Fl* | 5'-$NH_2$-CTTCATCCTGCTGCTATGCCTCATCTTCTTATTGG-3' |
| E | Biotin | 5'-$NH_2$-AGGGAAACTTAGAGTTGCCTTGAGCAGGAGTCGTG-3' |
| F | Biotin | 5'-$NH_2$-GCGGTATAAAGGGACTCACGATGCTGTACAGACTT-3' |
| G | none | 5'-TTGTCCTGGTTATCGCTGGATGTGTCTGCGGCGTT-3' |

*Where Fl = fluorescein.

Where fluorescein is the member of a reactive pair (ie. primers C and D), sense primers (5'-$NH_2$-TTGTCCTGGTTATCGCTGGATGTGTCTGCGGCGTT-3'and 5'-$NH_2$-CTTCATCCTGCTGCTATGCCTCATCT-TCTTATTGG-3') were treated with 2 mg of fluorescein isothiocyanate in 100 $\mu$L of sodium borate buffer, pH 9.0, at room temperature for 15 hours. The excess fluorescein was removed by passage of the reaction mixture through a NAP-5 column (Pharmacia, Piscataway NJ) which had been equilibrated with 10 mL of water. The solvent was removed from the eluate in vacuo, and the yellow residue was taken up into 150 $\mu$L formamide. The fluoresceinated oligonucleotide was purified by gel electrophoresis (12% polyacrylamide/8M urea) on a slab gel (0.15 x 40 cm) at 40 Watts constant power. After identification of the fluoresceinated band by UV shadowing, the primer was excised and extracted from the gel using 0.1 M TRIS, 0.5 M NaCl, 5 mM EDTA, at 60 °C for 16 hours.

Where biotin is the member of a reactive pair: (ie. primers E and F), antisense primers (5'-$NH_2$-AGGGAAACTTAGAGTTGCCTTGAGCAGGAGTCGTG-3' and 5'-$NH_2$-GCGGTATAAAGGGACTCACGATGCT-GTACAGACTT-3') were dissolved in 100 $\mu$L of 0.1 M sodium phosphate buffer, pH 7.5, and treated with 2 mg biotin-(aminocaproyl)$_2$-N-hydroxysuccinimide ester in 100 $\mu$L dimethylformamide (DMF) for 16 hours at room temperature. These primers were purified by gel filtration and electrophoresis as described above for the fluorescein-labeled oligonucleotides.

**Example 3: A Single Pair of Capture Primers**

Two reactions containing 1 $\mu$L of a DNA solution with either 0 or $10^5$ hepatitis B virus (HBV) plasmid DNA molecules per $\mu$L was used as a target for 30 cycles of PCR with primers D and F (see Table **1**, as prepared in example **2** with reactive pair members) in a buffer containing 10mM Tris pH 8.3, 50 mM KC1, 2.5mM $MgC1_2$, 200 $\mu$M of each deoxyribonucleotide triphosphate (dNTP), 0.01% gelatin, and 1.25 units of $T_{aq}$ DNA polymerase. The final oligonucleotide concentration was 0.1 $\mu$M each. Each sample was overlayed with a small volume of mineral oil. Samples were heat denatured at 94 °C for 5 minutes prior to cycling. Each PCR cycle consisted of a 1 min. denaturation step at 94 °C; a 2 min, annealing step at 50 °C; and a 2 min. extension step at 72 °C. Reaction volume was 50 $\mu$L. The amplified DNA product was 392 base pairs

(bp) as determined by the physical location of the two primer sequences within the HBV surface antigen gene. The double labeled DNA products with reactive pair members at each end were detected by a microparticle immunoassay performed on a prototype of the Abbott **IM$_x$**® system using anti-fluorescein coated microparticles and an anti-biotin:alkaline phosphatase detection conjugate. The result was:

| No. of molecules | Signal |
|---|---|
| 0 | 51.0 |
| $10^5$ | 323.8 |

**Example 4: Nested Pairs of Primary and Secondary Primers**

Six reactions containing 1µL of a stock DNA solution with either 0, $10^2$, $10^3$, $10^4$, $10^5$, $10^6$ HBV plasmid DNA molecules per µL was used as a target for 30 cycles of PCR using the HBV surface antigen gene specific primary oligonucleotide primers A and B. PCR reaction conditions were identical to those described in Example **3** except that the final oligonucleotide concentration was 1 µM each. The amplified DNA product was 492 base pair (bp) as determined by the physical location of the two primer sequences within the HBV surface antigen gene.

After completing 30 PCR cycles, 5 µL of each reaction was used as target for an additional 5 cycles of PCR (50 µL total reaction volume) using the HBV surface antigen gene specific oligonucleotide primers C and E (see Table **1**, prepared as in Example **2** with reactive pair members) which hybridize with the target at a site adjacent (but not overlapping) the site where the **3'** terminus of the primary primers A and B would be if hybridized to the same strands. The final oligonucleotide concentration was 1 µM each. The 203 bp PCR products with reactive pair members at each end of the double-stranded DNA molecules were detected by a microparticle immunoassay performed on a prototype of the Abbott **IM$_x$**® system using anti-fluorescein coated microparticles and an anti-biotin:alkaline phospatase detection.conjugate. Results were:

| No. of molecules | Signal |
|---|---|
| 0 | 8.2 |
| $10^2$ | 11.6 |
| $10^3$ | 106.7 |
| $10^4$ | 611.8 |
| $10^5$ | 984.2 |
| $10^6$ | 1100.4 |

**Example 5: Single Nested Capture Primer**

Two reactions containing 1 µL of a DNA solution with either 0 or $10^5$ HBV plasmid DNA molecules per µL was used as a target for 30 cycles of PCR with HBV surface antigen gene specific primary oligonucleotide primers A and F (See Table **1**), primer F being prepared as in Example **2** with a single reactive pair member. Final oligonucleotide concentration was 1 µM each. PCR reaction conditions were identical to those described in Example 4 above.

After completing 30 PCR cycles, 5 µL of each reaction was used as a target for an additional 5 cycles of PCR using the HBV surface antigen gene specific oligonucleotide primers C and F (see Table **1**, prepared as in Example **2** with reactive pair members). The final oligonucleotide concentration was 1 µM each. The 440 bp double labeled DNA products were detected as described in Example 2. The result was:

| No. of molecules | Signal |
|---|---|
| 0 | 36.2 |
| $10^5$ | 225.3 |

**Example 6: Directly Labeled Primers**

Synthesis of direct label

4-(2-carboxy-1-ethyl)-7-hydroxy-2-oxo-2H-1-Benzopyran was synthesized according to the procedure diagrammed below, (through structure 5).

In each of the following cases, R represents Hydrogen.

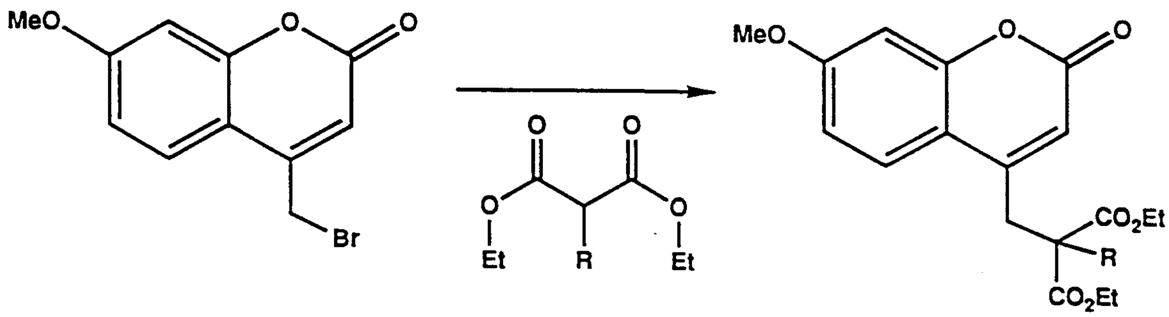

2

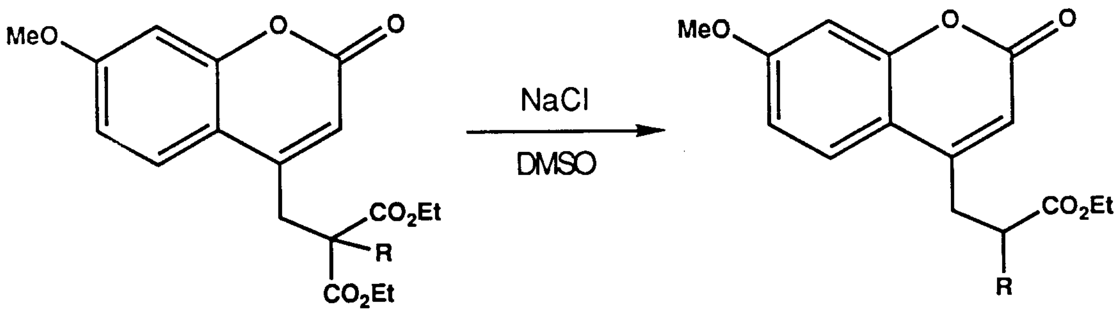


2 3

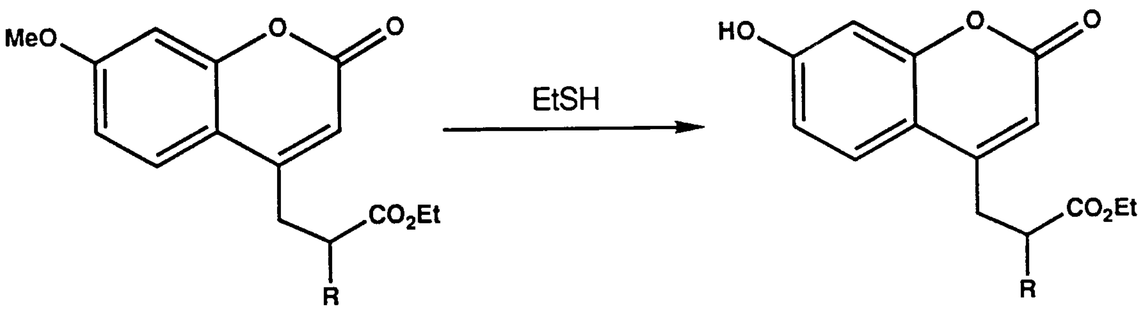


3 4

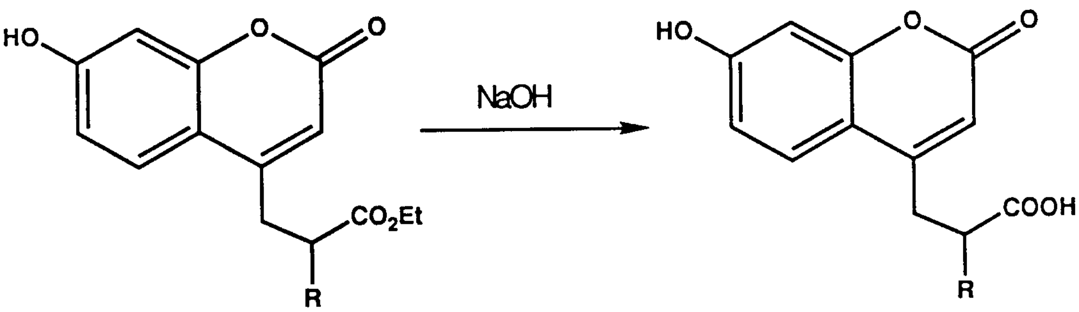


4 5

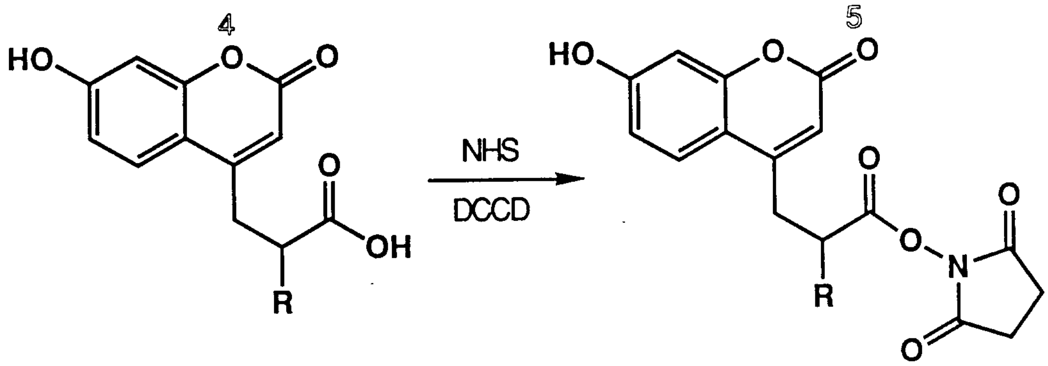


5 6

Briefly, 4-bromomethyl-7-methoxycoumarin (1) (Aldrich Chemicals) was converted to 4-(2-bis-(carbethoxy)-1-ethyl)-7-methoxy-2-oxo-2H-1-Benzopyran (2) as follows.

To a suspension of 240 mg of 60% NaH mineral oil dispersion (6 mmol) in 10 mL of DMF was added 961 $\mu$L (6mmol) of diethyl malonate. After the foaming subsided and the suspension cleared to a solution, 1346 mg (5mmol) of 4-bromomethyl-7-methoxycoumarin (1) was added all at once. After stirring for 4 h at room temperature, the DMF was stripped off, and the residue partitioned between 0.01 M HCl/hexane. The organic phase was concentrated and vacuum dried, then as much as possible was taken up into 4 mL of 50/50 EtOAc/hexane.

To a solution of 44.8 mg (0.13 mmol) of 4-(2-bis(carbethoxy)-1-ethyl)-7-methoxy-2-oxo-2H-1-Benzopyran (2) in 6 mL of DMSO was added 15 mg of NaCl, followed by 4.6 mL of water. The reaction was stirred in an oil bath at 180 °C for 2.5 h, and was cooled to room temperature. After addition of 45 mL of water to the reaction mixture, the resultant emulsion was extracted with 2x40 mL EtOAc. The organic phase was concentrated by rotary evaporation, and was vacuum dried. After uptake into 3 mL of 25% EtOAc in hexane, flash chromatography using the same solvent system gave 31.4 mg of 4-(2-carbethoxy-1- ethyl)-7-methoxy-2-oxo-2H-1-Benzopyran (3), 88%.

To a suspension of 726 mg (5.4 mmol) of $AlCl_3$ in 10 mL of dichloromethane at 0 °C was added 4 mL of EtSH. The suspension became a clear solution within seconds. Then, 298 mg (1.08 mmol) of (3) in 4 mL of dichloromethane was added, turning the yellow solution red in color. The ice bath was removed, and the reaction stirred to room temperature for 3 h. The solvents were removed in vacuo, and the residue thoroughly vacuum dried. The residue was extracted into EtOAc as much as possible, then the extract was flash chromatographed using 30/70 EtOAc/hexane. The long- and short-wave UV active band gave 76.7 mg (27%)of 4-(2-carbethoxy-1-ethyl)-7-hydroxy-2-oxo-2H-1-Benzopyran (4)

A 36.7 mg sample of ester (4)was suspended into 10 mL of water, and 25 mL of 50% aqueous NaOH was added. The resultant solution was stirred at room temperature for 4 h. TLC analysis (30/70 EtOAc/hexane) after this time showed that no starting material remained. The mixture was acidified using 1 mL of 1 M HCl. A precipitate formed upon acidification, and the white solid was left to deposit for 1 h. After the solid was filtered off and thoroughly washed with 1 M HCl, it was vacuum dried to give 14.1 mg (43%) of analytically pure 4-(2-carboxy-1-ethyl)-7-hydroxy-2-oxo-2H-1-Benzopyran (5).

Direct Labeling of Primers

4-(2-carboxy-1-ethyl)-7-hydroxy-2-oxo-2H-1-Benzopyran (5) was activated to 4-(2-carboxy-1-ethyl)-7-hydroxy-2-oxo-2H-1-Benzopyran, N-hydroxy succinimide ester (6). as follows: To a suspension of (5) in 6 mL of MeCN was added 4.7 mg (41 mmol) of N-hydroxysuccinimide, 8.4 mg (41 mmol) of dicyclohexylcarbodiimide (DCCD) and 2 mg of 4,4-dimethylaminopyridine.The rection as stirred at room temperature for 24 h, and the solvent was removed in vacuo. The residue was taken up into 750 µL of DMF. Thereafter, (6) was directly coupled with the antisense PCR primer, 5'-$NH_2$-GCGGTATAAAGGGACTCACGATGCTG-TACAGACTT-3'. The resulting labeled oligonucleotide was purified, in 14% yield, in identical fashion to the primers containing a member of a reactive pair (See Example **2** above).

PCR and Detection of Labeled Primers

PCR was run using 100 pmol of each primer and 40 attomoles (*i.e.* 40 x $10^{-18}$ mole) of HBV target. After addition of 0.5 units Taq polymerase and 2 nmol of the essential nucleotide triphosphates to a total volume of 100 µL, the reaction was cycled for 1 min at 95 °C (denature), 2 min at 50 °C (anneal), and 2 min at 74 °C (primer extension). After 30 cycles, the microfuge tubes were left at 4 °C. Agarose gel electrophoresis showed that the PCR generated a double-stranded product DNA band. With no added target, the PCR did not proceed.

The automated detection of double labeled PCR product was accomplished using antifluorescein-coated latex microparticles for capture and fluorescence detection as the readout. The antifluorescein was covalently coupled to carboxylated latex microparticles using EDAC. The fluorescence detection of microparticle-captured, coumarin-labeled, double-stranded product was done on a breadboard version of the **$IM_x$**® analyzer (Abbott Laboratories, IL) with modified software. The coumarin-labeled PCR primer possessed an excitation maximum at 378 nm, with an emission maximum at 460 nm. These maxima are well within the wavelength detection ranges for the **$IM_x$**®. The protocol for the automated capture/fluorescence readout on the instrument is as follows: A 35 µL sample of PCR product was diluted to 100 µL using ammonium carbonate buffer at pH 9. Then, 35 µL of solution was incubated with 75 µL of antifluorescein-linked microparticles (0.1% solid v/v) at 37 °C for 10 min. After dispensing 100 µL of the incubation solution onto a fiberglass matrix and washing with ammonium carbonate buffer at pH 9, the signal on the microbeads was spectrophotometically determined and recorded as:

| PCR Product | counts |
|---|---|
| Fluorescein-Coumarin | 417 |
| Fluorescein-Biotin | 388 |
| Fluorescein-No label | 379 |

**NOTE:** The units of the signal in this example are counts, because the coumarin label is always present and is not generated by an enzyme. In all previous cases, the signal units are counts/sec/sec, which is a rate. This is because the fluorescent moiety was generated by alkaline phosphatase, and it is the rate of this enzyme which is determined.

### Example 7 Non-Instrument Detection

Four different double-stranded PCR products, produced as in Example 3, were presented for detection by a microparticle immunoassay procedure. One PCR product contained no haptens (derived from primers B and G); another contained only fluorescein hapten (derived from primers B and C); a third contained only biotin hapten (derived from primers F and G); and the fourth contained both a fluorescein and a biotin hapten (derived from primers C and F). These PCR products were incubated with anti-fluorescein coated microparticles and were captured on **$IM_x$**® wedges. Anti-biotin:alkaline phosphatase conjugate was added, and bound product was visualized by the addition of nitro blue tetrazolium/5-bromo-4-chloro-3-indolyl phosphate. Referring to Figure 6, visible color is present only in the bottom right wedge, which contains PCR products labelled with both biotin and fluorescein.

### Example 8 Immunochromatographic Detection

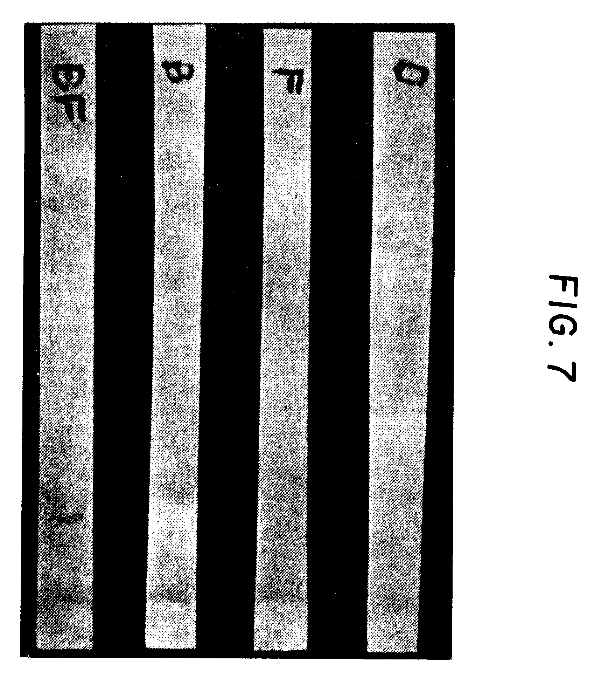

Four different double-stranded PCR products, produced as in Example 7, were presented for detection by an immunochromatographic technique as taught in publication EP 299 428. The PCR products were incubated with a suspension comprising anti-biotin-conjugated colloidal selenium dispersed in a solution of 150 mM NaCl, 100 mM TRIS, 2% alkali-treated casein, pH 7.4, for about 10 seconds. Into this suspension was dipped a strip of nitrocellulose (approx. 0.4 x 4.8 cm) onto which had been spotted, approx. 1 cm from the lower end, anti-fluorescein at a concentration of 0.2 mg/mL. As capillary action caused the suspension to migrate up the strip, the anti-biotin:selenium complex was retained on the strip only when both biotin and fluorescein were present on the PCR product (See Figure 7).

### Example 9 Spectrophotometric Detection

Three different PCR products, produced as in Example 3, were presented for detection by a solid-phase enzyme immunoassay system using anti-fluorescein coated 6 mm (1/4 inch) polystyrene beads and an anti-biotin:alkaline phosphatase conjugate. One of these PCR products contained only biotin hapten; another only fluorescein hapten; and the third both biotin and fluorescein. Signal was measured using a **Quantum**® spectrophotometer (Abbott Laboratories).

| PCR product | Signal |
|---:|---|
| Biotin only | 0.133 |
| Fluorescein only | 0.161 |
| Biotin and Fluorescein | 0.565 |

## Claims

1. A method for amplifying and detecting a target nucleic acid sequence in a sample, comprising:
   a) hybridizing to each of the target and its complementary strand respective oligonucleotide primers, one of which is conjugated to a member of a reactive pair and the other of which is conjugated to or capable of reacting with a detectable moiety;
   b) synthesizing from each primer an extension product complementary to each nucleic acid strand, the extension product of each primer being used in the synthesis of extension products from the other primer once the extension product is separated from its complement;
   c) separating the primer extension products from their complements to produce single-stranded molecules;
   d) treating the single-stranded molecules generated from step (c) with the primers of step (a) under conditions that a primer extension product is synthesized using each of the single strands produced in step (c) to form double-stranded products;
   e) capturing said double-stranded products from step (d) on a solid phase having associated therewith the other member of said reactive pair so that said double-stranded products become linked to said solid phase by said reactive pair; and
   f) detecting said detectable moiety associated with said solid phase.

2. The method of claim 1 wherein the other primer of step (a) is directly conjugated to a detectable moiety.

3. The method of claim 1 wherein the other primer of step (a) is conjugated to a first member of a second reactive pair, the second member of said second reactive pair being conjugated to a detectable moiety, and wherein prior to step (f) the method further comprises treating said double stranded products with said second member of said second reactive pair.

4. The method of claims 1 or 3 wherein said reactive pairs are selected from the group of antibody and antigen, biotin and avidin, enzyme and enzyme receptor, carbohydrate and lectin, biotin and antibiotin, and pairs of complementary DNA strands.

5. A method for further amplifying and detecting amplified, double stranded target nucleic acid sequences in a sample, comprising:
   a) hybridizing to the complementary amplified target strands a pair of secondary oligonucleotide primers, each of said secondary primers being linked to first members of different reactive pairs;
   b) synthesizing from each secondary primer extension products complementary to the target nucleic acid strands, the extension product of each primer being used in the synthesis of extension products from the other primer once the extension product is separated from its complement;
   c) separating the primer extension products from their complements to produce single-stranded molecules;
   d) treating the single-stranded molecules from step (c) with the primers of step (a) under conditions that a primer extension product is synthesized using the single strands in step (c) to form double-stranded products;
   e) capturing said double-stranded products from step (d) on a solid phase having associated therewith a second member of one of said reactive pairs, so that said double stranded products become linked to said solid phase by said reactive pair; and
   f) before, during or after step (e), treating said double-stranded products of step (d) with detectable second members of the other of said reactive pair, said second members being reactive with the other of said first reactive members on said double-stranded products to link therewith; and
   g) detecting the detectable second members associated with said solid phase to indicate whether amplified products of the target strand are present.

6. The method of claim 5 wherein said first amplification is performed by PCR using a pair of primary oligonucleotide primers.

7. The method of claims 1 or 5 wherein steps (c) and (d) are repeated at least once.

8. The method of claims 3 or 5 wherein said reactive pairs are different.

9. The method of claims 3 or 5 wherein each of said primers is linked at its 5' end to its reactive pair member.

10. A method for amplifying and detecting target oligonucleotide sequences in a sample where for each target sequence two primary primers are employed, one to hybridize with the target sequence and the other to hybridize with its complement, to form primary extension products complementary to the target and its complement, the primary extension products being used in the synthesis of further primary extension products from the primary primers, wherein at least one of the two primary primers is linked to a group comprising a first member of a first reactive pair or to a labeling group selected from: i) a detectable label and ii) a first member of a second reactive pair; said method comprising:

    during or after the reaction of the primary primers and target sequence, treating the primary extension products with at least one additional secondary primer which can hybridize with the primary extension product of the linked primary primer at a site 3' to the linked primary primer's terminus to form double-stranded secondary extension products, said secondary primer being linked to the other of said group comprising a first member of a reactive pair and said labeling group;

    capturing double-stranded secondary extension products on a solid phase having associated therewith the other member of said first reactive pair so that said double-stranded products become associated with said solid phase by said reactive pair; and

before, during or after the capturing step, optionally reacting the double-stranded secondary extension products with a detectable label conjugated to the second member of said second reactive pair; and

determining the detectable label associated with said solid phase.

**11.** The method of claim 10 wherein said primary extension products are treated with two secondary primers.

**12.** The method of claim 10 or 11 wherein said first and second reactive pairs are different.

**13.** The method of claims 10 or 11 wherein each of said linked primers is linked at its 5' end to its reactive pair member.

**14.** The method of claim 10 wherein said label group is a member of a second reactive pair.

**15.** The method of claim 10 wherein said label group is a directly detectable moiety.

**16.** The method of claims 10 or 11 wherein said specific binding pairs are selected from the group consisting of antibody and antigen, biotin and avidin, enzyme and enzyme receptor, carbohydrate and lectin, biotin and antibiotin, and pairs of complementary DNA strands.

**17.** A kit for amplifying and detecting a target nucleic acid sequence, comprising:

at least two primers, one primer complementary to said target sequence, and the other primer complementary to the target-complementary sequence wherein primer extension products can be synthesized from said primers such that each extension product forms a template for the synthesis of further extension products from the primers and wherein at least one of said primers has a reactive pair member linked to it and the other of said primers is linked to a reactive pair member capable of reacting with a detectable moiety or to a detectable label;

a DNA polymerase; and

a solid phase having linked thereto a member of a reactive pair which can bind to said reactive pair member on said one primer.

**18.** The kit as recited in claim 17 wherein the other primer is linked to a first member of a second reactive pair.

**19.** The kit of claim 18 further including a conjugate which includes a detectable moiety linked to the second member of said second reactive pair.

**Patentansprüche**

**1.** Verfahren für die Vervielfachung und den Nachweis einer Zielnukleinsäuresequenz in einer Probe, umfassend:

a) Hybridisierung jeder Zielsequenz und ihres jeweiligen Komplementärstranges mit Oligonucleotid-Primern, von denen einer an ein Glied eines Reaktandenpaares konjugiert ist und der andere an eine nachweisbare Gruppe konjugiert, oder aber in der Lage ist, mit dieser zu reagieren,

b) Synthese eines Verlängerungsproduktes von jedem Primer, das komplementär zu jedem Nucleinsäurestrang ist, wobei das Verlängerungsprodukt eines jeden Primers für die Synthese von Verlängerungsprodukten des anderen Primers verwendet wird, sobald das Verlängerungsprodukt von seinem Komplement abgetrennt ist,

c) Trennen der Verlängerungsprodukte der Primer von ihren Komplementen zur Erzeugung von Einzelstrangmolekülen,

d) Behandeln der in Schritt (c) erzeugten Einzelstrangmoleküle mit den Primern von Schritt (a) unter solchen Bedingungen, daß ein Verlängerungsprodukt eines Primers synthetisiert wird unter Verwendung der in Schritt (c) erzeugten Einzelstränge zur Bildung von Doppelstrangprodukten,

e) Verankern der Doppelstrangprodukte aus Schritt (d) an einer festen Phase, die das andere Glied des reaktiven Paars trägt, so daß das Doppelstrangprodukt mittels dieses reaktiven Paars an die feste Phase gebunden wird und

f) Nachweis der nachweisbaren Gruppe, die mit der festen Phase assoziiert ist.

2. Verfahren nach Anspruch 1, wobei der andere Primer aus Schritt (a) direkt an eine nachweisbare Komponente konjugiert ist.

3. Verfahren nach Anspruch 1, wobei der andere Primer aus Schritt (a) an das erste Glied eines zweiten Reaktandenpaares konjugiert ist, und wobei das zweite Glied des zweiten Reaktandenpaares an eine nachweisbare Gruppe konjugiert ist, und wobei vor Schritt (f) das Verfahren darüberhinaus die Behandlung der Doppelstrangprodukte mit dem zweiten Glied des zweiten Reaktandenpaares umfaßt.

4. Verfahren nach Anspruch 1 oder 3, wobei die Reaktandenpaare aus der Gruppe Antikörper und Antigen, Biotin und Avidin, Enzym und Enzymrezeptor, Kohlenhydrat und Lectin, Biotin und Antibiotin, und aus Paaren komplementärer DNS-Stränge gewählt sind.

5. Verfahren für die weitere Vervielfachung und zum Nachweis von vervielfachten doppelsträngigen Zielnucleinsäuresequenzen in einer Probe, umfassend:
   a) Hybridisierung eines Paars sekundärer Oligonucleotid-Primer mit den komplementären vervielfachten Zielsequenzsträngen, wobei jeder sekundäre Primer mit ersten Gliedern unterschiedlicher Reaktandenpaare verbunden ist,
   b) Synthese von Verlängerungsprodukten ausgehend von jedem sekundären Primer, wobei die Verlängerungsprodukte komplementär zu den Zielnucleinsäuresträngen sind, und wobei das Verlängerungsprodukt jedes Primers zur Synthese von Verlängerungsprodukten des anderen Primers verwendet wird, sobald das Verlängerungsprodukt von seinem Komplement abgetrennt ist,
   c) Trennen der Verlängerungsprodukte der Primer von ihren Komplementen zur Erzeugung von Einzelstrangmolekülen,
   d) Behandeln der in Schritt (c) erzeugten Einzelstrangmoleküle mit den Primern aus Schritt (a) unter solchen Bedingungen, daß ein Verlängerungsprodukt eines Primers unter Verwendung der in Schritt (c) erzeugten Einzelstränge unter Bildung von Doppelstrangprodukten synthetisiert wird,
   e) Verankern der Doppelstrangprodukte aus Schritt (d) an einer festen Phase, an die das zweite Glied eines der Reaktandenpaare assoziiert ist, so daß das Doppelstrangprodukt über dieses Reaktandenpaar an die feste Phase gebunden wird, und
   f) vor, während oder nach Schritt (e) die Behandlung der Doppelstrangprodukte aus Schritt (d) mit nachweisbaren zweiten Gliedern des anderen Reaktandenpaares, wobei die zweiten Glieder mit den ersten reaktiven Glieder auf diesen Doppelstrangprodukten unter Bindung an diese reagieren, und
   g) Nachweis der nachweisbaren zweiten Glieder, die an die feste Phase assoziiert sind, um anzuzeigen ob vervielfachte Produkte der Zielstränge vorhanden sind.

6. Verfahren nach Anspruch 5, wobei die erste Vervielfachung durch PCR unter Verwendung eines Paars primärer Oligonucleotid-Primer erhalten wird.

7. Verfahren nach Anspruch 1 oder 5, wobei die Schritte (c) und (d) mindestens einmal wiederholt werden.

8. Verfahren nach Anspruch 3 oder 5, wobei die Reaktandenpaare unterschiedlich sind.

9. Verfahren nach Anspruch 3 oder 5, wobei jeder Primer an seinem 5'-Ende mit einem Glied seines Reaktandenpaares verbunden ist.

10. Verfahren zur Vervielfachung und zum Nachweis von Zieloligonucleotidsequenzen in einer Probe, wobei für jede Zielsequenz zwei primäre Primer eingesetzt werden, von denen einer mit der Zielsequenz und der andere mit deren Komplement hybridisiert, dies unter Bildung primärer Verlängerungsprodukte komplementär zur Zielsequenz und zu deren Komplement, wobei die primären Verlängerungsprodukte zur Synthese weiterer primärer Verlängerungsprodukte von den primären Primern verwendet werden, wobei mindestens einer der beiden primären Primer an eine Gruppe umfassend ein erstes Glied eines ersten reaktiven Paars oder an eine Markierungsgruppe, gewählt aus: i) einer nachweisbaren Markierung und ii) einem ersten Glied eines zweiten Reaktandenpaares gebunden ist, wobei das Verfahren umfaßt:
    Behandeln der ersten Verlängerungsprodukte während oder nach der Reaktion der primären Primer mit der Zielsequenz mit mindestens einem weiteren sekundären Primer, der mit dem primären Verlängerungsprodukt des gebundenen primären Primers an einer 3'-Stelle am gebundenen primären Primer-Ende hybridisieren kann, unter Bildung doppelsträngiger sekundärer Verlängerungsprodukte,

wobei der sekundäre Primer an das andere Glied der Gruppe bestehend aus erstem Glied eines Reaktandenpaares und einer Markierungsgruppe gebunden ist,
Verankern der doppelsträngigen sekundären Verlängerungsprodukte an eine feste Phase, an die das andere Glied des ersten reaktiven Paars assoziiert ist, so daß die Doppelstrangprodukte mit der festen Phase über das reaktive Paar assoziiert werden, und
vor, während und nach dem Verankerungsschritt optionale Umsetzung der doppelsträngigen sekundären Verlängerungsprodukte mit einer nachweisbaren Markierung, die an das zweite Glied des zweiten Reaktandenpaars konjugiert ist, und
Bestimmen der nachweisbaren Markierung, die mit der festen Phase assoziiert ist.

**11.** Verfahren nach Anspruch 10, wobei die primären Verlängerungsprodukte mit zwei sekundären Primern behandelt werden.

**12.** Verfahren nach Anspruch 10 oder 11, wobei das erste und zweite Reaktandenpaar verschieden sind.

**13.** Verfahren nach Anspruch 10 oder 11, wobei jeder der gebundenen Primer an seinem 5'-Ende mit einem Glied seines reaktiven Paars verbunden ist.

**14.** Verfahren nach Anspruch 10, wobei die Markierungsgruppe ein Glied des zweiten Reaktandenpaares ist.

**15.** Verfahren nach Anspruch 10, wobei die Markierungsgruppe eine direkt nachweisbare Komponente ist.

**16.** Verfahren nach Anspruch 10 oder 11, wobei die spezifischen Bindungspaare aus der Gruppe bestehend aus Antikörper und Antigen, Biotin und Avidin, Enzym und Enzymrezeptor, Kohlenhydrat und Lectin, Biotin und Antibiotin, und Paaren komplementärer DNS-Stränge gewählt werden.

**17.** Ein Kit für die Vervielfachung und den Nachweis einer Zielnucleinsäuresequenz, umfassend:
mindestens zwei Primer, wobei ein Primer komplementär zu der Zielsequenz und der andere komplementär zu der Zielsequenz-komplementären Sequenz ist, worin Verlängerungsprodukte von den Primern so synthetisiert werden können, daß jedes Verlängerungsprodukt eine Matrize für die Synthese weiterer Verlängerungsprodukte der Primer bildet, wobei mindestens an einen der Primer ein Glied eines Reaktandenpaares gebunden ist und wobei der andere Primer an ein Glied eines Reaktandenpaares gebunden ist, das in der Lage ist, mit einer nachweisbaren Gruppe oder nachweisbaren Markierung zu reagieren,
eine DNS-Polymerase, und
eine feste Phase, an die ein Glied eines reaktiven Paars gebunden ist, das an das Glied des Reaktandenpaares an einem Primer binden kann.

**18.** Das Kit nach Anspruch 17, wobei der andere Primer an ein Glied eines zweiten Reaktandenpaares gebunden ist.

**19.** Das Kit nach Anspruch 18, zu dem desweiteren ein Konjugat gehört, das eine nachweisbare Gruppe enthält, die an das zweite Glied des zweiten Reaktandenpaares gebunden ist.

## Revendications

**1.** Procédé d'amplification et de détection d'une séquence d'acides nucléiques cible dans un échantillon comprenant :
a) l'hybridation sur la cible et sur son brin complémentaire d'amorces respectives d'oligonucléotides, dont l'une est conjuguée à un membre d'une paire réactive et dont l'autre est conjuguée à, ou capable de réagir avec, un fragment détectable ;
b) la synthèse à partir de chacune des amorces d'un produit d'extension complémentaire de chacun des brins d'acide nucléique, le produit d'extension de chacune des amorces étant utilisé dans la synthèse de produits d'extension à partir de l'autre amorce une fois le produit d'extension séparé de son complément ;
c) la séparation des produits d'extension des amorces de leurs compléments pour produire des molécules simple brin ;

d) le traitement des molécules simple brin générées dans l'étape (c) avec les amorces de l'étape (a) dans des conditions telles qu'un produit d'extension d'amorce soit synthétisé en utilisant chacun des simples brins produits dans l'étape (c) pour former des produits double brin ;
e) la capture desdits produits double brin de l'étape (d) sur une phase solide ayant associée à elle l'autre membre de ladite paire réactive de telle sorte que lesdits produits double brin se lient à ladite phase solide par ladite paire réactive ; et
f) la détection dudit fragment détectable associé à ladite phase solide.

**2.** Procédé de la revendication 1 dans lequel l'autre amorce de l'étape (a) est directement conjuguée à un fragment détectable.

**3.** Procédé de la revendication 1 dans lequel l'autre amorce de l'étape (a) est conjuguée à un premier membre d'une seconde paire réactive, le second membre de ladite seconde paire réactive étant conjugué à un fragment détectable, et dans lequel, avant l'étape (f), le procédé comprend en outre le traitement desdits produits double brin avec ledit second membre de ladite seconde paire réactive.

**4.** Procédé de la revendication 1 ou de la revendication 3, dans lequel lesdites paires réactives sont sélectionnées dans le groupe des anticorps et des antigènes, de la biotine et de l'avidine, des enzymes et des récepteurs d'enzyme, des hydrates de carbone et de la lectine, de la biotine et de l'antibiotine et des paires de brins d'ADN complémentaires.

**5.** Procédé d'amplification supplémentaire et de détection des séquences d'acides nucléiques double brin cibles amplifiées dans un échantillon, comprenant :
a) l'hybridation sur les brins complémentaires amplifiés cibles d'une paire d'amorces oligonucléotidiques secondaires, chacune desdites amorces secondaires étant liée aux premiers membres de différentes paires réactives ;
b) la synthèse à partir de chacune des amorces secondaires de produits d'extension complémentaires des brins d'acide nucléique cibles, le produit d'extension de chacune des amorces étant utilisé dans la synthèse de produits d'extension à partir de l'autre amorce une fois le produit d'extension séparé de son complément ;
c) la séparation des produits d'extension des amorces de leurs compléments pour produire des molécules simple brin ;
d) le traitement des molécules simple brin générées dans l'étape (c) avec les amorces de l'étape (a) dans des conditions telles qu'un produit d'extension d'amorce soit synthétisé en utilisant les simples brins produits dans l'étape (c) pour former des produits double brin ;
e) la capture desdits produits double brin de l'étape (d) sur une phase solide ayant associée à elle le second membre de l'une desdites paires réactives de telle sorte que lesdits produits double brin se lient à ladite phase solide par ladite paire réactive ; et
f) avant, pendant et après l'étape (e), le traitement desdits produits double brin produits de l'étape (d) avec les seconds membres détectables de l'autre desdites paires réactives, lesdit seconds membres étant réactifs avec l'autre desdits premiers membres réactifs sur lesdits produits double brin pour se fixer sur eux ; et
g) la détection des seconds membres détectables associé à ladite phase solide pour indiquer si les fragments amplifiés du brin cible sont présents.

**6.** Procédé de la revendication 5, dans lequel ladite amplification est réalisée par PCR en utilisant une paire d'amorces oligonucléotidiques primaires.

**7.** Procédé des revendications 1 ou 5, dans lequel les étapes (c) et (d) sont répétées au moins une fois.

**8.** Procédé des revendications 3 ou 5, dans lequel lesdites paires réactives sont différentes.

**9.** Procédé des revendications 3 ou 5 dans lequel chacune desdites amorces est liée par son extrémité 5' à son membre de paire réactive.

**10.** Procédé d'amplification et de détection de séquences d'oligonucléotides cibles dans un échantillon où pour chaque séquence cible deux amorces primaires sont employées, une pour s'hybrider à la séquence cible et l'autre pour s'hybrider à son complément, pour former des produits d'extension

primaire complémentaires de la cible et de son complément, les produits d'extension primaires étant utilisés dans la synthèse d'autres produits d'extension primaires à partir des amorces primaires, dans lequel au moins une des deux amorces primaires est liée à un groupe comprenant un premier membre d'une première paire réactive ou un groupe marqueur choisi parmi : i) un marqueur détectable et ii) un premier membre d'une seconde paire réactive ; ledit procédé comprenant :
pendant ou après la réaction des amorces primaires et de la séquence cible, le traitement des produits d'extension primaires avec au moins une amorce secondaire supplémentaire qui peut s'hybrider avec le produit d'extension primaire de l'amorce primaire liée, en un site 3' sur l'extrémité de l'amorce primaire liée, pour former des produits d'extension secondaires double brin, ladite amorce secondaire étant liée à l'autre dudit groupe comprenant un premier membre d'une paire réactive et ledit groupe marqueur ;
la capture des produits d'extension secondaires double brin sur une phase solide ayant associé à elle l'autre membre de ladite première paire réactive de telle sorte que lesdits produits double brin s'associent à la phase solide par ladite paire réactive ; et
avant, pendant ou après l'étape de capture, la réaction éventuelle des produits d'extension secondaires double brin avec un marqueur détectable conjugué au second membre de ladite seconde paire réactive ; et
la détermination du marqueur détectable associé à ladite phase solide.

**11.** Procédé de la revendication 10 dans lequel lesdits produits d'extension primaires sont traités avec deux amorces secondaires.

**12.** Procédé de la revendication 10 ou de la revendication 11, dans lequel lesdites première et seconde paires réactives sont différentes.

**13.** Procédés des revendications 10 ou 11, dans lequel chacune desdites amorces liées est liée par son extrémité 5' à son membre de paire réactive.

**14.** Procédé de la revendication 10, dans lequel ledit groupe marqueur est un membre d'une seconde paire réactive.

**15.** Procédé de la revendication 10, dans lequel ledit groupe marqueur est un fragment directement détectable.

**16.** Procédé des revendications 10 ou 11, dans lequel lesdites paires spécifiques se fixant sont sélectionnées dans le groupe constitué d'anticorps et d'antigènes de la biotine et de l'avidine, d'enzymes et de récepteurs d'enzyme, d'hydrates de carbone et de la lectine, de la biotine et de l'antibiotine et de paires de brins d'ADN complémentaires.

**17.** Kit d'amplification et de détection d'une séquence d'acides nucléiques cible, comprenant :
au moins deux amorces, une amorce complémentaire de ladite séquence cible, et l'autre amorce complémentaire de la séquence complémentaire de la cible, grâce auquel les produits d'extension primaires peuvent être synthétisés à partir desdites amorces de telle sorte que chaque produit d'extension forme une matrice pour la synthèse d'autres produits d'extension à partir des amorces et grâce auquel au moins une desdites amorces a un membre d'une paire réactive lié à elle et l'autre desdites amorces est liée à un membre de paire réactive capable de réagir avec un fragment détectable ou un marqueur détectable ;
une ADN polymérase ; et
une phase solide ayant liée à elle un membre d'une paire réactive qui peut se fixer au dit membre de paire réactive sur une desdites amorces.

**18.** Kit tel que décrit dans la revendication 17, dans lequel l'autre amorce est liée à un premier membre d'une seconde paire réactive.

**19.** Kit de la revendication 18 comprenant en outre un conjugué qui comprend un fragment détectable lié au second membre de ladite seconde paire réactive.

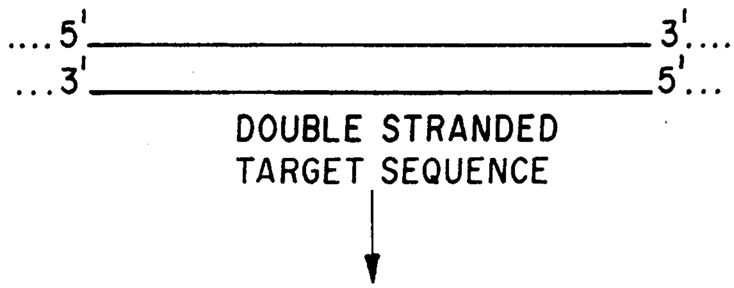


FIG.1A

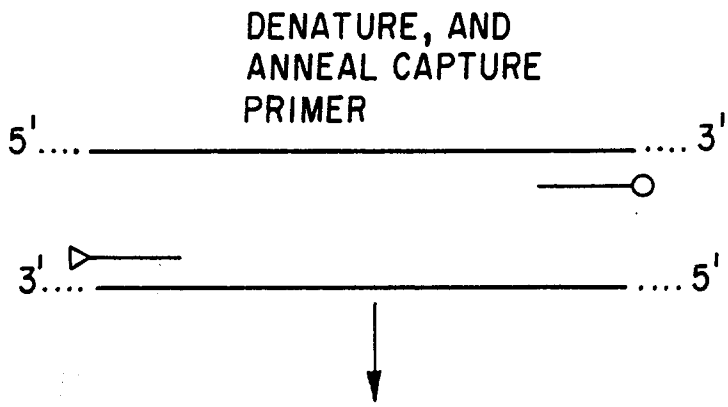


FIG.1B

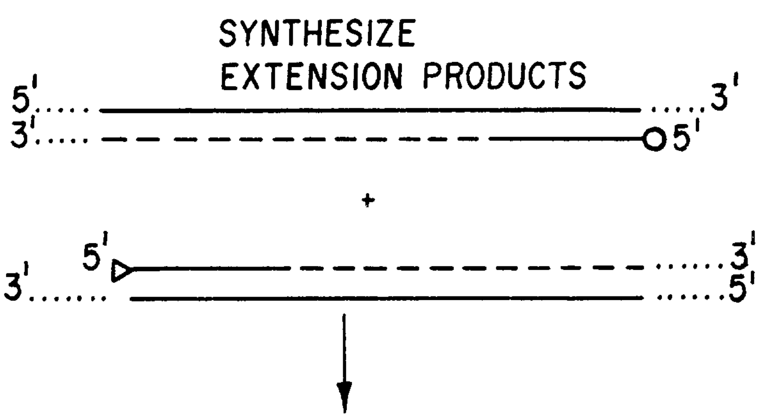


FIG.1C

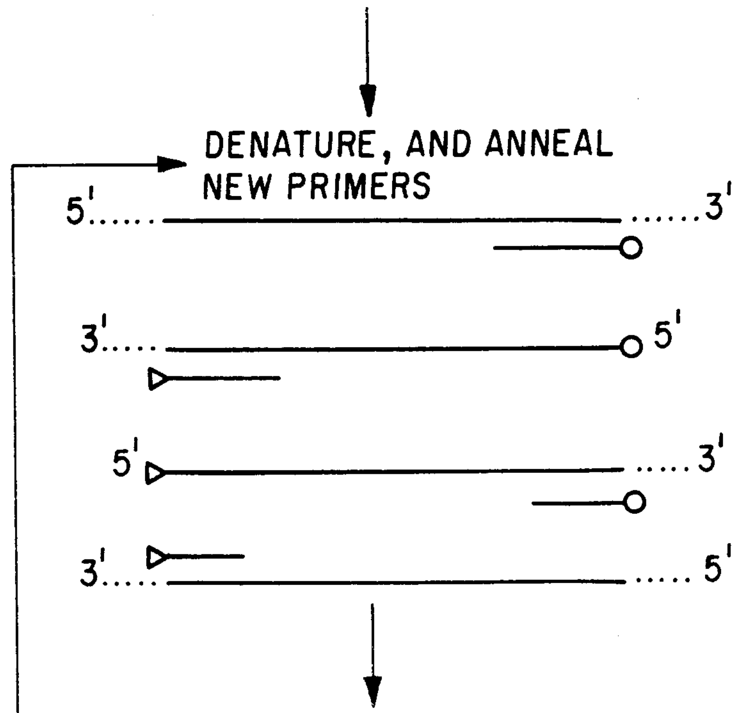
DENATURE, AND ANNEAL
NEW PRIMERS
5'
3'
3'
5'
5'
3'
3'
5'

FIG.1D

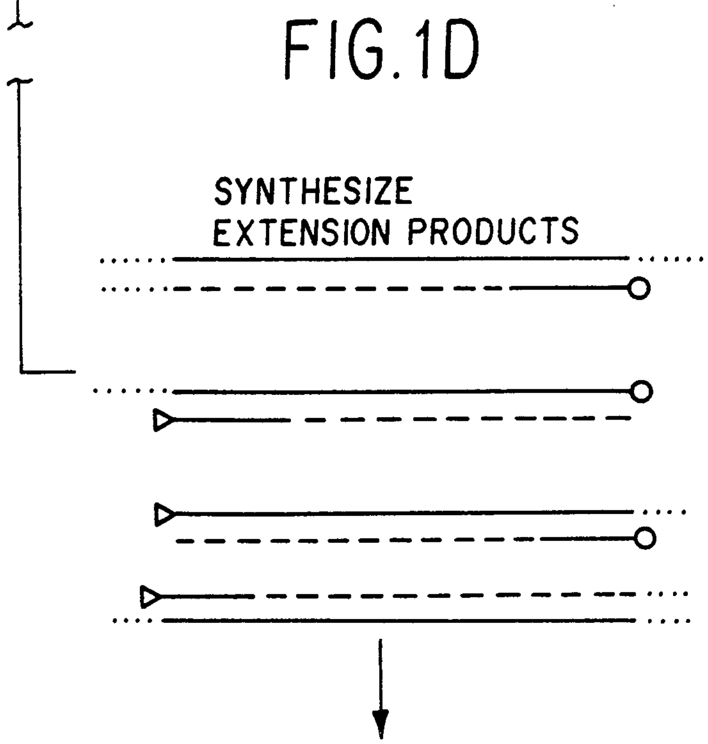
SYNTHESIZE
EXTENSION PRODUCTS

FIG.1E

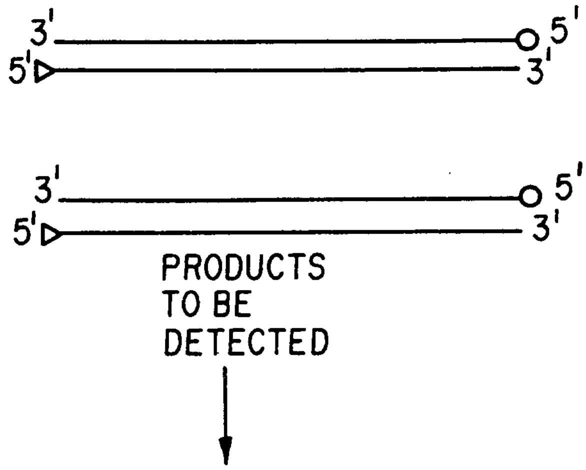


FIG.1F

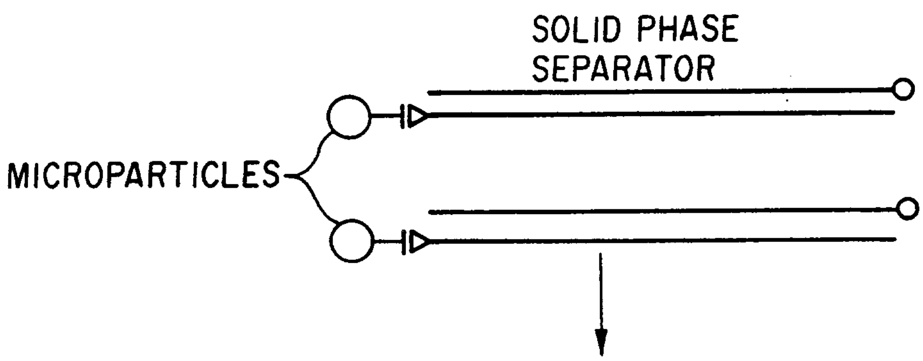


FIG.1G

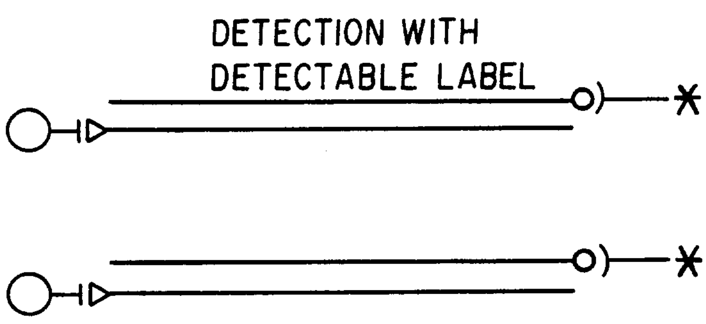


FIG.1H

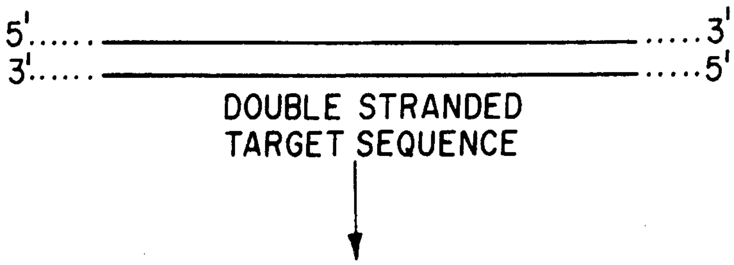


FIG. 2A

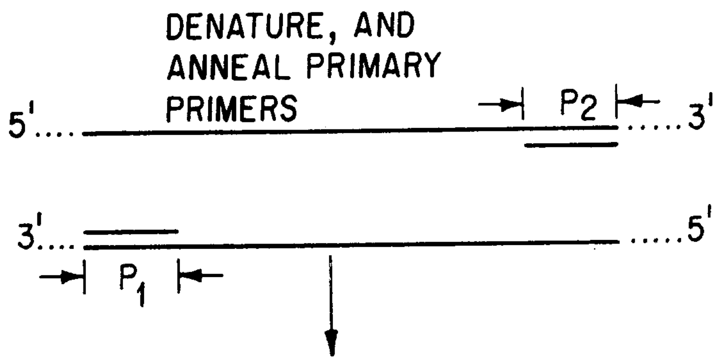


FIG. 2B

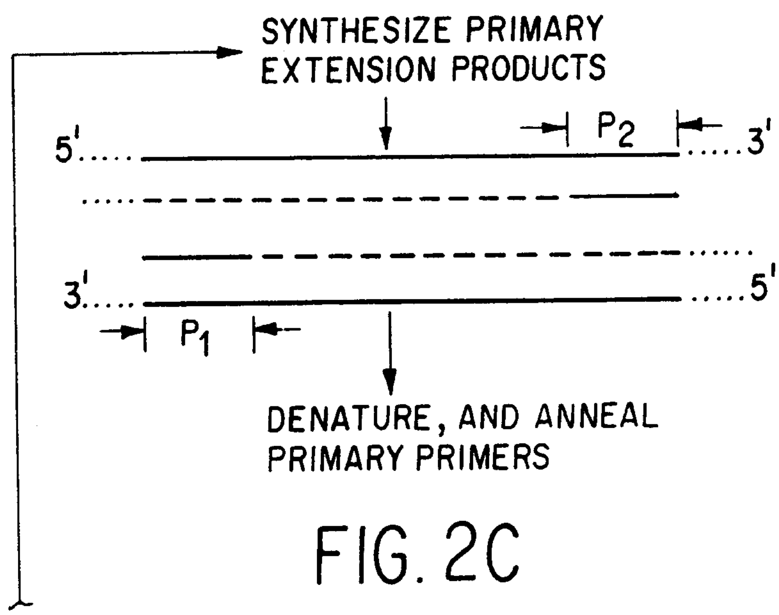


FIG. 2C

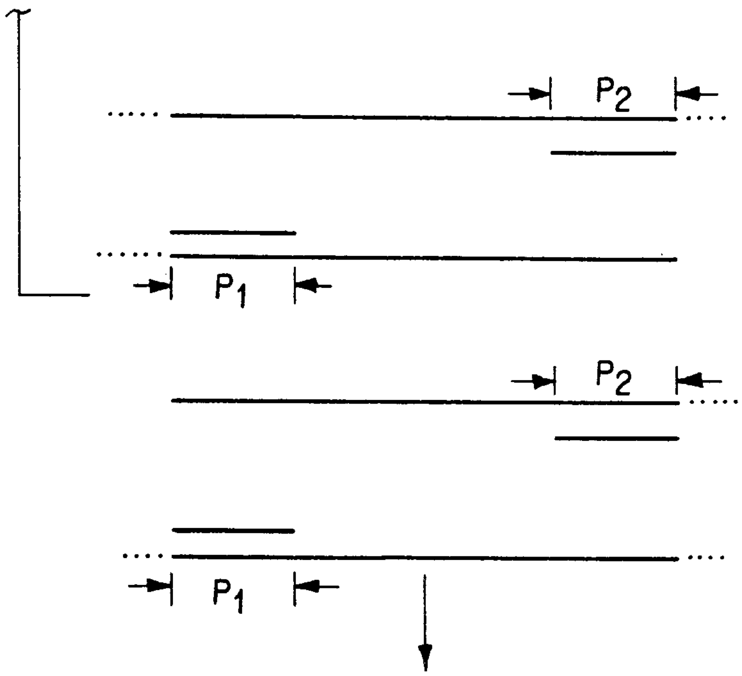


FIG. 2D

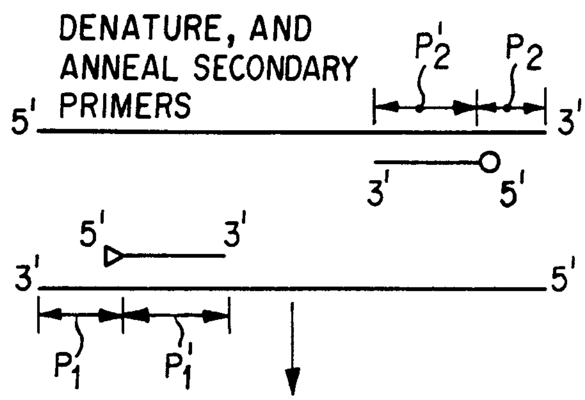


FIG. 2E

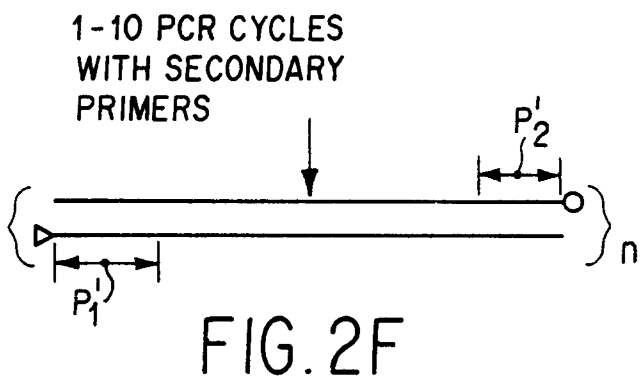


FIG. 2F

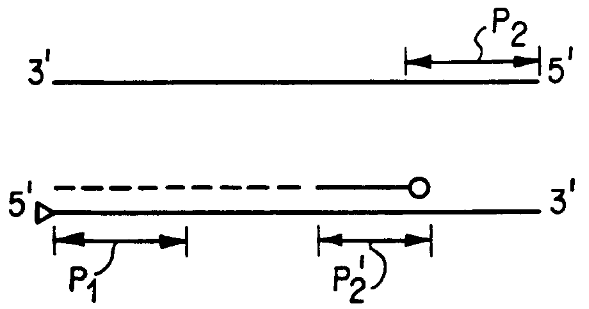


FIG. 3

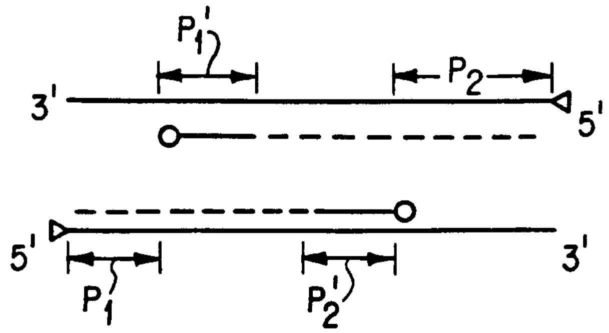


FIG. 4

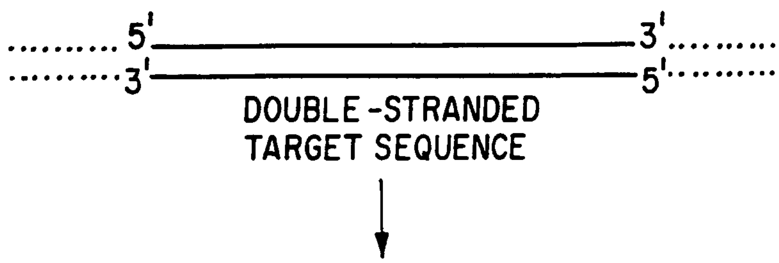


FIG. 5A

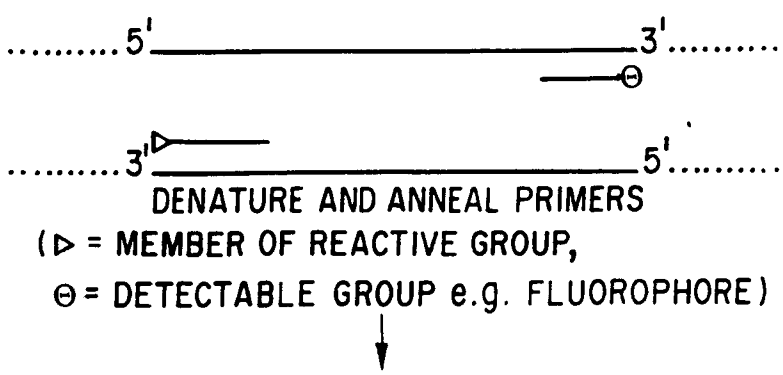


FIG. 5B

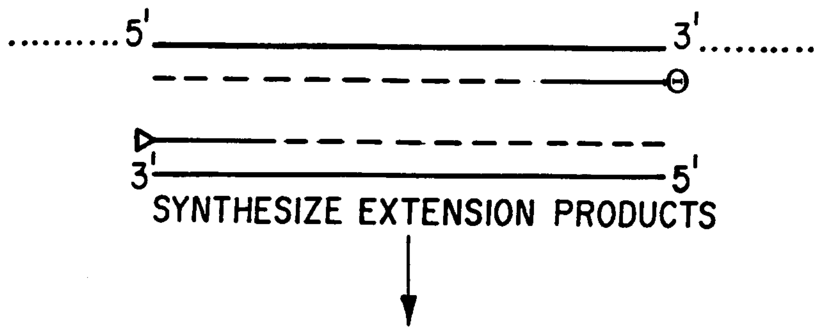


FIG. 5C

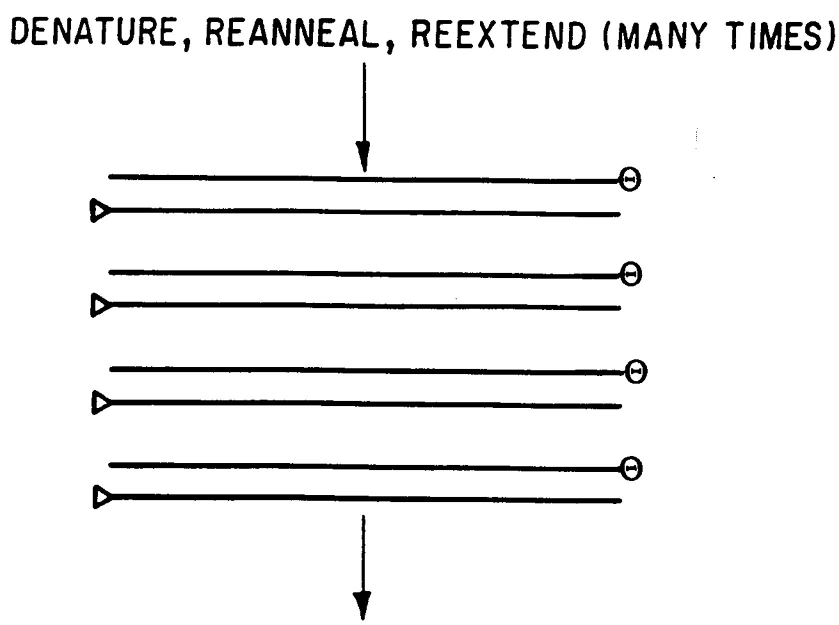


FIG. 5D

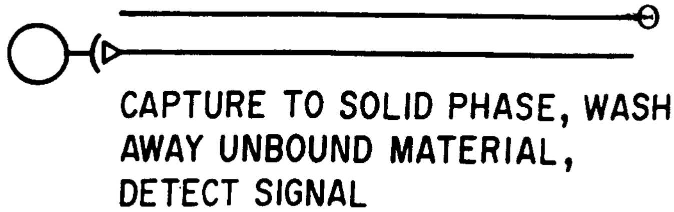


FIG. 5E

1 3

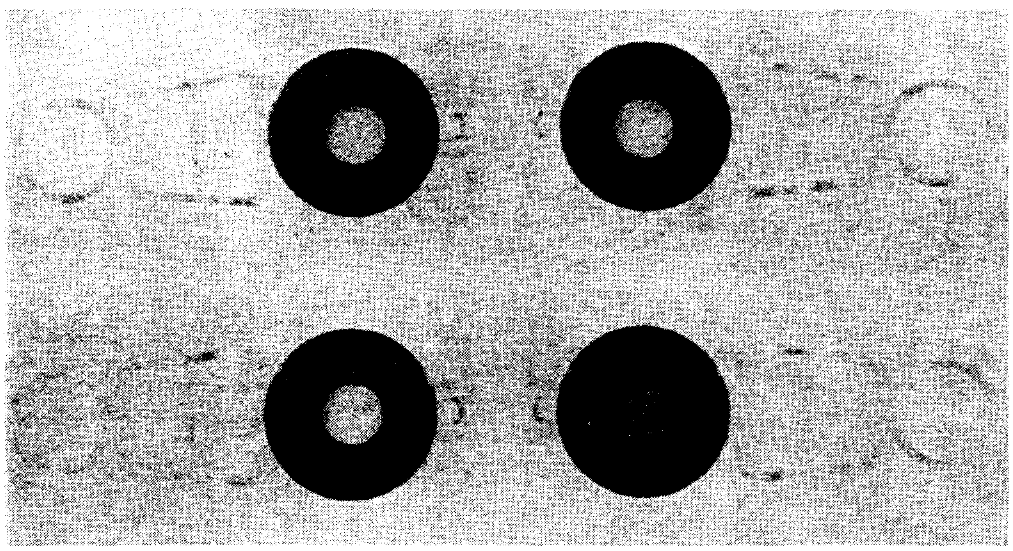

2 4

FIG. 6

FIG. 7